# EUROPEAN PATENT SPECIFICATION

(11) **EP 3 917 401 B1**
(45) Date of publication and mention of the grant of the patent: **15.10.2025**
(21) Application number: 20747891.8
(22) Date of filing: 23.01.2020
(51) Int. Cl.: A61B 5/20, G01N 21/82, G01N 31/02, G01N 33/493, G01N 33/84, G01N 33/52, A61B 5/145, A61B 5/1455

(54) **DEVICE AND METHOD FOR THE AUTOMATIC DETERMINATION OF ANION AND CATION LEVELS FROM URINE**
VORRICHTUNG UND VERFAHREN ZUR AUTOMATISCHEN BESTIMMUNG VON ANIONEN- UND KATIONENWERTEN AUS URIN
DISPOSITIF ET PROCÉDÉ DE DÉTERMINATION AUTOMATIQUE DES NIVEAUX D'ANION ET DE CATION À PARTIR D'URINE

(30) Priority: 28.01.2019 HU 1900027
(43) Date of publication of application: 08.12.2021
(73) Proprietor: IOI Auranae Kft., 1068 Budapest (HU)
(72) Inventor: KÕNTÕS, Zoltán, 1112 Budapest (HU)
(74) Representative: Szentpéteri, Zsolt
(86) International application number: PCT/HU2020/000003
(87) International publication number: WO 2020/157526

(56) References cited:
- WO-A1-2017/035882
- WO-A1-2018/122403
- CN-A- 101 787 730
- RU-A- 2003 134 132
- US-A- 4 766 080
- US-A1- 2007 071 648
- US-A1- 2017 022 536
- US-A1- 2017 322 197
- US-B2- 7 407 626

## Description

### BACKGROUND OF THE INVENTION

### 1. Technical Field of the invention

The invention relates to a device and a method for the automatic determination of anion and cation levels from urine. Urine analysis may provide a wide range of data of clinical significance not only regarding kidney function but also various metabolic processes, organ and cell system functions. The majority of the end products of metabolic processes are removed by the kidneys through urine.

Minerals are involved in the structure and the vital functions of the body. Almost all minerals are needed to build up the cells. A complete lack of minerals or lack of certain minerals will cause illness in the body, however, even they are found in traces in the nutrition, it will cover the functional needs. Minerals can be supplemented for the body in the form of food and sufficient amount of liquid (water), or in the form of nutritional supplements.

The mineral sodium is a cation of the body whose main function is to maintain the saltwater balance of the body. Sodium ions in the body are mainly present in the extracellular space, while the intracellular space contains a small amount of sodium ions. The mineral sodium is primarily excreted via the urine and this excretion is controlled by the body's needs (ADH hormone, aldosterone). Abnormal loss of the sodium minerals most likely to occur in adrenal or renal diseases. Abnormal accumulation of sodium minerals may develop in circulatory failure due to the weakness in the right part of the heart and in renal diseases with protein loss, which may lead to edema (swelling) after rapid increase in body weight.

The potassium ion, an ion exists in the body is the major cationic component of the intracellular space, while it is present only in very small amounts in the extracellular space. The levels of the Na⁺ and K⁺ and the ion exchange are closely related to each other, providing the proper osmotic pressure conditions between cells. The average daily potassium need of an adult human is 2-3 g (based on RDA-OÉTI), which is provided by balanced and mixed diet. Typical symptom of potassium deficiency is myasthenia and the intestinal atony (balky bowels). Extreme increases in potassium levels (for example, due to renal failure or excessive potassium intake) can lead to cardiac conduction disturbances (blocks), or even to cardiac arrest. Potassium is one of the macronutrients, i.e. is a mineral that the body needs in hundreds of milligrams or grams per day. Potassium plays an important role in maintaining fluid balance and gastro-intestinal tract balance, as well as in transmitting electrical impulses between nerve and muscle. It is of great importance that the transport of the hydrogen ions is also dependent on the presence of potassium, therefore, potassium plays an essential role also in maintaining the acid-base balance, as it can affect the changes of the pH in a favorable way for the body.

Calcium ion as a cation plays a key role in the function of many cells. Thus, for example, the calcium mineral plays a role in coupling of the stimulus and contraction in muscle cells, coupling of the stimulus and excretion in endocrine and exocrine glands and in blood coagulation. In addition to calcium, bones and the dentition also contain a large amounts of phosphate ions. It is found in the bones in the form of calcium phosphate. It also plays an important role in the energy-supply of the body, in the metabolism of the nutrients and in maintaining the acid-base balance.

The magnesium mineral as a cation contributes in the structure of teeth and bones in the body and plays a role in the transmission of stimuli, including nerve and muscle function. Magnesium is involved in about 300 different biochemical processes in the body. The amount of magnesium needed depends on a number of factors, such as age, gender, or whether a woman is pregnant or nursing. Magnesium plays a major role in the proper functioning of the muscles, inter alia by influencing the utilization of calcium and potassium. Magnesium also plays a major role in the proper functioning of cardiac muscles and neurotransmitting system. Depression, bad mood, lethargy, anxiety, nervousness and also hearing deficiency can also result from magnesium deficiency. The magnesium mineral is excreted mainly via the urine from the body.

Iodine is a mineral that becomes a constituent of thyroid hormones (as iodine), i.e. thyroxine and triiodothyronine and therefore plays an important role in metabolic processes, growth, and functioning of the nervous system, as thyroid hormones play a central role in the regulating cellular life and metabolism.

The primary role of selenium mineral in the body is the stabilization of the cell membrane and act as an antioxidant, i.e. it is effective in neutralizing free radicals. The lack of selenium minerals can cause skeletal muscle degeneration and muscle degeneration, resulting in disruption of the leg, improper gait and posture. The concentration of selenium minerals in the body can be detected in blood, urine, hair and nails. Selenium is an essential microelement with antioxidant activity and is a component of many enzymes. Lack of it can cause a variety of health problems in the function of the liver, thyroid, brain, testicles and ovaries.

The microelement lithium is very likely to influence a person's psychological mood. Lithium salts are used to treat manic depression (should not be given to a pregnant woman during pregnancy). Its deficiency affects the development of cardiac and circulatory diseases. Low intake reduces the reproductivity rate and affects the weight of the embryo.

The urine sample best reflects the body's recent mineral levels. Urine analysis is painless, and is one of the so-called non-invasive methods, and is therefore very common in everyday practice.

According to the present state of the art the rapid test and test strip applications are the most commonly used urine test methods for routine use, which methods are based typically on yes/no answers, i.e. are not suitable for quantitative analysis but for qualitative analysis only. Their function is based typically on the visible physiochemical changes resulting from the interaction with the component to be analyzed. The test stripe assay provides false positive and false negative results in high percentage. They are not capable of quantifying anions and cations.

Urine analysis can sometimes be time consuming and/or difficult to perform, if urinary stimulation is not present at the time of analysis. This is often why clinicians overlook this laboratory diagnostic method, which provides important information during clinical measures.

### 2. Description of the Prior Art

There are many solutions available in the prior art to assist with urine testing at home. Hungarian Patent No. 220777 relates to a solid state device for performing multi-analyte assays comprising a substrate and a multiple discrete reaction sites each containing a ligand covalently bonded to the substrate, wherein the surface of the substrate in the device is inert between the reaction sites with respect to analyte, and the analytes are selected from the group consisting of antibiotics, hormones, cardiac damage markers, infection markers, allergy markers, narcotics, enzymes, viruses, nucleotides and peptides. The samples to be analyzed in the device are whole blood, serum, plasma, urine, faeces, bile, tissue or nutritive. The multi-analyte analysing system comprises a device as disclosed above, a translation work surface, a sample dosing system, a liquid reagent delivery system, a tempered black box, a CCD camera and an image processing device. The disclosed solution can be used for simultaneous automatized detection of multiple substances, however, the compounds to be analyzed and the assay method differ from that of the present invention. Preparation of the substrate containing the biological ligands and analytic methodology thereof are extremely expensive and is not applicable for the determination of anion and cation levels and is most suitable for the detection of the presence of macromolecules.

International Publication No. WO 2018/122403 relates to a reusable device which is capable of quantifying at least one substance in the urine, such as a compound or a chemical component and the device can be mounted on the toilet bowl, especially on its rim. The device described includes a mounting part, a boxed electrical unit, an urine receiving area with at least one electrochemical sensor, a wireless connector capable of transmitting data to a remote central computer, wherein the electrochemical sensor comprises at least an ion sensitive Field Effect Transistor (ISFET) configured to be able to the detect the levels of one or more physiological ions. The obtained device is easy to use and easy to mount at home, even more than once. The electrochemical sensor can be set to measure the concentration of one or more substances selected from the group consisting of H⁺, Na⁺, K⁺, Ca²⁺, Mg²⁺, Cl⁻, H₂O₂, glucose, nitrites, proteins, estrogens, lutein hormone, beta-HCG hormone (hormone indicating pregnancy) and creatinine. The invention provides a solution for simple and rapid quantitative measurement of urine components at home, simple data processing and storage on IT devices. However, the disadvantage of the ISFET electrochemical sensor is that it can only be used within a narrow measuring range and therefore cannot be used to measure certain ions. In addition, for hygienic reasons, the attachable device is not suitable for multiple use. Conversely, frequent replacement of the device does not make the continuous monitoring cost effective on the basis of repeated measurements a day.

International Publication Nos. WO 2008/072112, WO 2017/160399 and WO 2017/058806 disclose urine analysis methods, however, their use relate to specific measurements and devices which are different from the solution of the present invention, they have limited applicability, and they are not suitable for cheap anion and cation level detection at home.

US2017022536 (A1) relates to a portable device for analyzing the presence of metabolites in urine, said device comprising a sampling unit, a reagent container, a unit for determining the quantity of metabolites and an electronic communication unit. The amount of the metabolites is measured by the color change after mixing the reagent with the urine sample. The analyte to be measured is glucose, nitrite, proteins, etc. The disclosed portable device can be mounted on the toilet bowl, thus, the hygienic problems mentioned above are present in this case as well, and the range of the metabolites to be analyzed do not cover the analytes that can be analyzed by the present invention, i.e. cations and anions.

CN 101 787 730 describes a smart toilet, which is suitable for taking samples from urine and performing various biochemical measurements.

US 7 407 626 describes the use of selective reagents during the analysis of urine.

The obvious disadvantage of the methods used so far is the ability to automate, that is, to perform serial measurements which give satisfactory results in quality, but the result is available in a much shorter time than at present. The cost of automated measurements is even lower than the cost of individual tests, so there is significant time, logistical and life-saving benefits of the proposed methods compared to currently available. A further advantage of the solution is that it allows the approximate determination of the amount of multiple anions and cations in the urine using a single device.

### SUMMARY OF THE INVENTION

It was an object of the present invention to provide a device for overcoming the above-mentioned problems which allows to sample from urine and to determine the level of anion and cation detectable in the urine in a hygienic way.

A further object was to provide a device capable of continuously monitoring the levels of a given anion and/or cation for several days through urine analysis, thereby providing an overview of the anion and/or cation supply of the body. Based on these measurements, the subject is able to decide which anion and/or cation needs additional intake or has to be abandoned. This allows the subject to prevent certain diseases associated with low or too high anion or cation levels in the body.

It has been recognized that there is an urgent need for a method and device for determining the anion and cation levels of urine in a rapid, yet relatively accurate measurement at home. Moreover, with proper use of modern devices, the values obtained allow continuous monitoring of the anion and cation levels of urine. The values thus obtained can assist in proper diagnosis of diseases by tracking inadequate amount of anion and cation for self-determining of the intake or abandonment of the corresponding anion and cation, thereby preventing the onset of certain diseases.

It has also been recognized that, due to its multi-purpose nature, up to 5 or 6 analysis can be performed on a single urine sample, depending on its volume, so that the proportion of individual anions and/or cations can be determined side by side. By measuring some cation and anion groups together, we can infer the physical state or the performance of the body.

The results of the analysis performed with the device can be shared electronically with experts, physicians, so a diagnosis can be made in a very short time without any travel waiting time and a possible intervention can be initiated. Intervention is also possible in places where so far, typically due to the duration of the analysis, it has not been possible to intervene meaningfully, such as in the case of athletes or heavy workers, since the device located at the workplace is capable of continuously monitoring the ion levels, thus maintaining the appropriate level of performance.

### DETAILED DESCRIPTION OF THE INVENTION

To solve the above objects, it has surprisingly been found that the determination of the amount of anions and cations can be accomplished by precipitation reactions that allow the reagent specific to a given ion to react selectively with only one component in a multiionic environment. The method is thus based on whether a particular anion or cation forms a precipitate with a reagent specific to it. The assay is carried out by adding said reagent to a suitably selected volume of urine until the selective precipitation begins, so that the amount of reagent is proportional to the concentration of the anion or cation dissolved in the urine.

For example, magnesium ions can be selectively detected in the form of hydroxide precipitate, well distinguished from the calcium ions, because under the conditions used, Mg(OH)₂ precipitates, while Ca(OH)₂ remains in solution, which is determined by their solubility. The amount of the reagent is proportional to the concentration of the Mg ions in the urine.

Calcium ions can be selectively detected in the form of a white oxalate precipitate, well distinguishable from other ions, since oxalate ions do not react with other ions under the conditions used, and thus, the low solubility of calcium oxalate allows the determination.

Potassium ions can be selectively detected in a precipitation reaction in the form of a yellow precipitate with sodium hexanitrito cobaltate[III] reagent, well distinguished from other ions.

Sodium ions can be selectively detected in a precipitation reaction in the form of a white precipitate with potassium hexahydroxy antimonate [V] reagent, well distinguished from other ions.

Lithium ions can be selectively detected in a precipitation reaction in the form of a white precipitate with potassium hydrogen phosphate reagent, well distinguished from other ions.

Iodide ions can be selectively detected in a precipitation reaction by reacting with IOI Blue^{®} 0,5 reagent (copper nitrate), well distinguished from other anions.

Selenate ions can be selectively detected in the form of a color reaction in a hydrochloric acid medium with thiourea reagent, well distinguished from other ions, giving a purple color reaction where the color depth is proportional to the concentration of the selenate ions.

Phosphate ions can be selectively detected in a precipitation reaction in the form of a white precipitate with lithium hydroxide reagent, well distinguished from other ions.

The basic criteria for performing each assay are 20 ml urine sample and 15 minutes assay time. Adjusted to the concentration of each ion to be tested, a set of reagents were selected to provide the precipitation phenomenon and detection from 20 ml urine in 15 minutes.

The invention relates to a device for automatic determination of anion and/or cation levels in urine, as defined in appended independent claim 1.

The device of the present invention measures the anion and cation levels of the urine, in particular Na⁺, K⁺, Ca⁺, Mg²⁺, I⁻, Se²⁻, Li⁺, PO₄³⁻ ions. The storage unit of the reagent solutions suitable for detecting the anion and cation levels by selective precipitation reaction contains in particular the following reagent solutions: calcium hydroxide, oxalate salts, sodium hexanitritocobaltate[III], potassium hexahydroxy antimonate[V], potassium hydrogen phosphate, copper nitrate, thiourea dissolved in hydrochloric acid medium, and lithium hydroxide. Each of these reagent solutions can be used to detect a particular anion or cation.

The invention further relates to a device suitable for the testing one or even more anions and/or cations or other parameters simultaneously.

The present invention relates to a device wherein the turbidity sensor is connected to the sample storage compartment through a horizontal and a vertical sample forwarding unit, which, together with sample storage compartment, are designed to be controlled by a logic control, said logic control includes the plurality of IT elements computing the test results (such as the number of drops of the specific reagent solution) and sets of units transmitting the said test results to the unit for collecting and transmitting the data.

The present invention relates to a device wherein the sampling unit comprising a flowmeter with sensor for separating 1 to 100 ml, preferably 10 to 20 ml of urine sample to the sample holder. The sampling unit is designed to be capable of sampling one or more samples.

The invention further relates to a device wherein the sampling unit comprises at least one electromechanical unit for delivering the sample holders in horizontal and vertical direction. In the device of the present invention the reagent solution is added into the sampling vessel via an element having a dropping needle.

The present invention relates to a device further comprising, within its active range, an adjustable inner parameter storing unit and a built-in radio frequency communication transceiver for the detection of the proximity of IT devices, preferably smart devices having the similarly configured parameters and an ioFIT identifier.

The present invention further relates to a device further comprising a radio frequency transceiver, an antenna, a mechanical unit for fixing the said device, wherein the active range of the radio frequency transceiver can be variably set to specific values, and the inner parameter storage unit has a fix selection template preset to the parameters stored in it, wherein said selection template can be broadened or modified.

The invention further relates to a device having a digital accounting system associated with measurements for offsetting each test service in which, when a measurement is made, the transaction value in internal accounting unit [Rz] is subtracted from the internal accounting units [Rz] of the smart device having the same ioFIT identifier.

The present invention further relates to a device which further comprising a smart contract storage unit wherein by accepting the said smart contract, after sending the measured parameter and recording said measured parameter from the informatic device with ioFIT identifier the unit automatically charges a predetermined internal accounting unit on the internal account having the ioFIT identifier identical with the ioFIT identifier of the informatic device from which the urine test request was sent and thereby the agreement will be executed.

The invention further relates to the use of a device for the automatic determination of anion and/or cation levels in urine, as defined in appended independent claim 12. By analyzing of successive urine samples, it allows continuous monitoring of specific anion and/or cation levels for several days, thus providing an overview of the measured anion and/or cation supply in the body. Based on these measurements the subject can decide which cation and/or anion needs additional intake or, in case of high levels, to leave foods containing that cation and/or anion in their diet. This allows the subject to prevent certain diseases associated either low or too high anion and/or cation levels in the body.

Preferred embodiments of the urine testing device of the present invention will now be described in more detail with reference to the accompanying Figures.

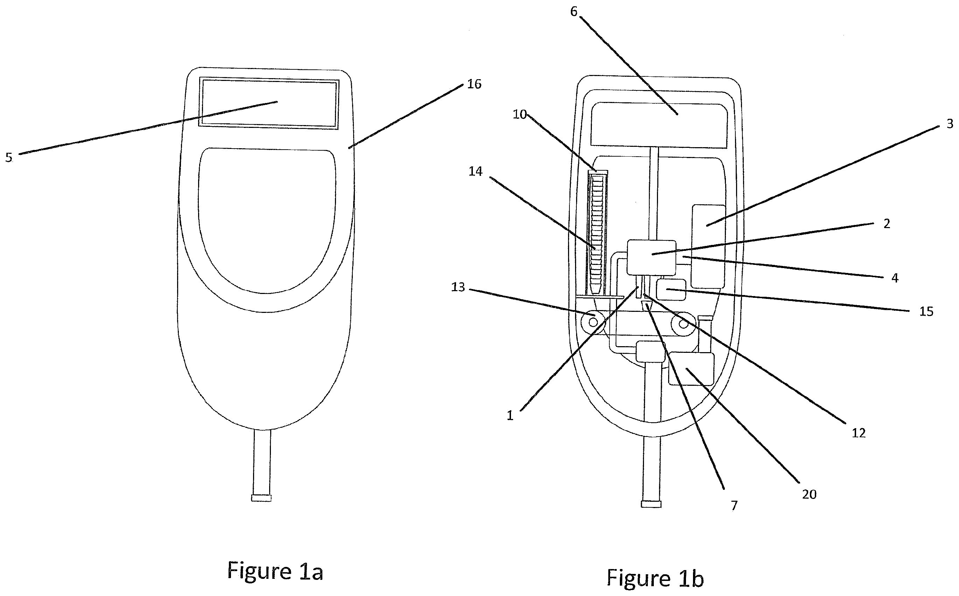

Figure 1a. shows the front view of the automated device for the detection and analysis of individual urine cations and anions, and Figure 1b. shows the internal structure of the urinal version of the device.

### EXAMPLES

### Example 1. Automatic anion and cation level detection in urine

The device for carrying out the urine analysis method by precipitation according to the present invention, comprises inter alia the following components:
a) at least one sampling unit 1,
b) at least one automated turbidity measuring unit 2,
c) at least one reagent solution storage unit 3 for the selective precipitation reaction,
d) at least one pump 4 for delivering the reagent solution suitable for the precipitation reaction of the ion to be detected,
e) at least one human machine interface (HMI) 5,
f) at least one unit for collecting and transmitting data 6, and
g) at least one central control 15 for controlling the process,
which components are arranged in a properly designed frame 16.

During the method for the automated analysis of cations and anions [ions] of the urine, the ions from the urine are analyzed in an automated turbidity measuring unit 2 connected to at least one sampling unit 1, such that the aqueous solutions of metal ions are used as analytical reagent, wherein said solutions are added automatically and dropwise to the sample to be analyzed with the pump 4, wherein the appearance of precipitates forming upon interaction of said solution with urine indicate the end point of the analysis/detection. When the end point is reached, the number of reagent liquid droplets indicates the result of the measurement, which is processed digitally stored and electronically by the unit for collecting and transmitting data 6. The measurement data is transmitted by the unit for collecting and transmitting data 6 to the IT device having ioFIT identifier of the subject, said IT device is subjected to identification and authorization steps prior to the sampling step. The sample containing sampling vessel 7 is connected directly or indirectly to the turbidity measuring unit 2 containing the measuring instrument, while the measuring instrument is connected to the unit for collecting and transmitting data 6.

In one aspect of the present invention, there is provided a method of detecting and analyzing particular urine cations and anions comprising measuring the parameters, preferably quantitative parameters, of the ionic components from a urine sample. In a further aspect, further parameters can also be determined during the method. In addition to the at least one automated measuring unit 2 suitable for measuring turbidity, additional measuring instruments may be included to determine additional parameters in the method. Examples of such urine analyzing methods include, but are not limited to, methods for measuring urine temperature and pH. For the automated turbidity measuring unit 2, UV and IR detection methods, among others, wherein the infrared [IR] detection methods are suitable for detection of carboxyl group containing organic anions, among others, and the ultraviolet detection methods are suitable for detection of gold complexes. The turbidity detector may be complemented by, but not limited to, IR and UV detectors.

The essence of the method of the present invention is to prepare the particular urine cations and anions [ions] for the automated analysis and measurement in aqueous solution by sampling directly from the urine [if necessary, through a suitably selected filter surface] by transferring it into a sampling vessel 7 using a suitably selected valve, said sampling vessel 7 is designed to have an opening-closing element on its upper surface, preferably an inlet valve and an opening-closing inlet element having reagent-dosing inlet, preferably having an inlet valve which can be sealed tightly. According to one aspect of the device for analyzing urine samples is suitable for sampling a clean [free from suspended substances] urine sample, wherein the sampling vessel 7 is sealed by a cap, wherein said vessel can preferably be filled or emptied without removing the cap.

In one aspect the invention relates to a method for automated turbidity detection of a sample by automatically adding a reagent solution to a sample until its turbidity reaches a predetermined value, which is the end of the detection. The drop number of the reagent added to the sample is proportional to the amount of the ion to be detected.

The device according to the invention comprises a reagent solution storage unit 3 and dispensing unit, reagent dosing valves, a turbidity measuring unit 2 comprising a sensor capable of measuring turbidity during reagent dosing in a given volume of sample in the sample compartment, and a unit for data collecting and transmitting 6, wherein these units are suitably arranged in a housing 16 suitable for urination. In the device, the turbidity sensor is connected to the sample vessel storage unit 10 through the horizontal and vertical sample forwarding unit 13,14, which are arranged with the sample vessel storage unit 10 under common logical control, which comprising a set of the IT elements for computing the measurement result (the drop number the corresponding reagent solution), and elements which collecting and transmitting the results to the unit for collecting and transmitting data 6.

In the sampling vessel storage unit 10 comprised by the device according to the invention there are sampling vessels 7 are arranged, which are rotated by a stepping mechanism. The sampling vessels 7 are preferably cylindrical and have a positioning element. The sampling vessel storage unit 10 comprises at least one electromechanical unit for forwarding the sampling vessel 7 horizontally and vertically. The stepping mechanism may move freely through a vertical sample forwarding unit 14. The sampling vessel storage unit 10 further comprises a horizontal sample forwarding unit 13 by means of which the collected urine sample is placed horizontally in various positions for measurements towards measuring instruments for performing the selected measurements and finally towards the disposal vessel 20.

The reagent solution is introduced into the sampling vessel 7 via element provided with dropping needle 12. The sampling unit 1 includes a flow sensor and flow meter for separating 20 ml of urine sample into the sampling vessel 7. The sampling unit 1 further includes a flow direction changing valve for disposing the excess urine after the 20 ml of urine sample is transferred into the sampling vessel 7. The control unit 15 is coupled with a distance sensor arranged outside the frame or separated from the frame in order to detect the subject who would like to perform the measurement and accordingly, to activate the HMI 5 interface panel.

The determination of the cation and anion levels of the urine sample is performed by an electronically controlled turbidity measuring unit 2 which detects the end points of the precipitation reached by dropwise addition of a reagent solution to the sample which sample has a predetermined volume and originates from the urine to be tested, said reagent solution is delivered by pump 4, and the cation and anion levels are determined by means of detecting the turbidity generated in the sample having a predetermined volume. The device according to the invention comprises a sample analysing system, wherein the sampling vessel 7 containing the urine sample are arranged so that they move horizontally from the last position of the sample vessel storage to sample analysis site and after the analysis it is delivered to disposal vessel 20. The sampling vessels 7 are preferably cylindrical and have a positioning element. The stepping mechanism is free to move horizontally.

The pump 4 is a material delivering unit connecting the sampling vessel 7 with the reagent solution storage unit 3.

Turbidity is measured by a turbidity measuring detector unit. The device according to the invention further comprises a unit for data collecting and transmitting 6, which includes a horizontal sample forwarding unit 13, a vertical sample forwarding unit 14 and the controlling ports of the unit for data collecting and transmitting 6, which are connected to the central control unit 15.

In another aspect of the invention, the device may include further measuring instruments in addition to at least one turbidity measuring unit 2. These include, but are not limited to, UV detectors or IR detectors which are used to measure additional physical and/or chemical properties of the urine. These measuring instruments are also properly connected to the central control unit 15 and to the unit for collecting and transmitting data 6.

The device according to the invention comprises within its active range an adjustable inner parameter storing unit capable of active communication, an integrated radio frequency communication transceiver for the detecting the proximity of IT devices, preferably smart devices having similarly adjusted parameters and having an ioFIT identifier, said device further comprises a 6 unit for collecting and transmitting data and at least one human machine interface (HMI) 5 and have an individual device identifier [MID]. The device according to the invention further comprises a power source, electronic memory chip and a radio frequency communication transceiver, antenna, a mechanical fixing unit and a housing 16 enclosing them. The active range of the radio frequency communication transceiver can be variably set to specific values and the inner parameter storage unit comprises a preset, scalable and modifiable, fix selection template for the parameters stored therein.

The human machine interface (HMI) 5 unit of the device according to the invention may also include a proximity sensor that turns on the device in the vicinity of the subject intending to perform the analysis.

The device according to the invention further comprises a unit for collecting and transmitting data 6 which is suitable for collecting reagent drop number data and/or other urine physical parameter data [temperature, conductivity], wherein the unit for collecting and transmitting data 6 comprises a processing unit for processing the data it collects, the automated dropper connects to said unit for collecting and transmitting data, and a display unit, as well as, a unit for transmitting the reagent solution drop number data and/or the urine physical parameter data to the unit for collecting and transmitting data 6 and/or to the display or to the IT device of the subject in a suitable displayable form for the subject, a primer data collecting module for monitoring reagent drop number data provided by the automatic dropper and/or a second data collecting module for collecting other urine physical parameter data [temperature, conductivity], and/or a third data collecting module for monitoring the usage data of the whole device.

The device according to the invention is provided with a dual function structure for operability by which in the first step analyses the urine sample and in the second step transmits results to the nearby IT device, preferably a smart device having an ioFIT identifier by unit for collecting and transmitting data 6, while an auto-executable smart contract stored on the mass storage device is activated and the value of the above transaction for the analysis in internal accounting unit [Rz] is subtracted from the internal accounting units [Rz] belonging to the IT device, preferably a smart device having the same ioFIT identifier. The value of the above transaction will be charged in internal accounting units [Rz] to the account of the IT device having the same MID identifier.

The present invention also relates to the implementation of a digital coin insertion in connection with device and analyzing method, according to the principle of the present coin inserting devices whereby, if an internal accounting unit account belonging to a specific ioFIT identifier is electronically chargeable and the authorizing instruction is issued [before executing the smart contract] the cost of the analysis may be offset with Rz internal accounting unit which corresponds to the "insertion" of an electronic coin.

According to another approach of the invention, the measurement data and the payment and accounting processes may be stored in a blockchain structure.

During the implementation of the present invention, it automatically performs predefined method steps, such as sampling, analysis, rinsing, result transmitting. It is a multifunctional application since it is able to measure multiple cations and anions in one procedure.

In another aspect of the present invention, the device also comprises a housing 16 enclosing an interior space and being capable for displaying an interactive advertisement, said housing 16 includes electronic and non-electronic advertising space on it. In a further aspect of the present invention the device also includes a central control unit 15 arranged inside the housing 16 wherein the central control unit 15 has one or more communication ports for signal transmission and at least one communication port for the central control unit 15 is in connection with the unit for collecting and transmitting data 6. This part of the device according to the present invention may contain advertisements and may be used for advertising purposes.

The device of the present invention also includes a smart contract on a storage unit, and when a request is received from the IT device with an ioFIT identifier for a urine test to the device with a specific MID identifier, upon transmitting the parameter [drop number] presenting the result of the analysis to the IT device with the ioFIT identifier [simultaneously saving the data in the IT device], the account of the same ioFIT identifier is automatically debited with an [Rz] internal accounting unit amount predefined in the smart contract [which will be credited to the MID account of the party performing the measurement], and thereby the contract is executed. The subject of the contract is the analysis to be performed and the settlement of its offset in internal accounting units according to the fee schedule. The contracting parties are the owner of the IT device with the ioFIT identifier, preferably a smart device and the business organization operating the devices according to the invention.

Different embodiments of the invention may be provided with different designs. Thus, for example, the design of the housing 16 may differ from that urinal design shown on Figures 1a. and 1b. In another aspect of the device according to the present invention can be used as a conventional toilet solution regardless of gender, while the urinal design is male-only. Of course, we do not intend to limit the scope of the invention to the following embodiments, so that further embodiments and arrangements within the scope of the invention may be provided.

In the following, a preferred embodiment of the device and method of the present invention will be described in detail, but it is not intended to limit the scope of the invention to the embodiment illustrated herein.

### Example 2. Detection of iodide ions in urine with Cu(NO₃)₂ solution by means of a urinal design of the device of the invention

A subject with smart device having an ioFIT identifier requiring the automatic determination of [iodide ion] anion and cation levels in urine was initiated the detection by selecting the corresponding ion(s) to be analyzed via the human machine interface (HMI) 5 on the device housing or via a suitable IT device, preferably on a smart device, through a suitable data transmitting connection. 20 ml of urine was sampled and transferred to the sampling vessel 7 by the sampling unit 1 inside the device of the present invention. The subject with a smart device having an ioFIT identifier was detected by the proximity sensor of the HMI 5.

Inside the sampling unit 1 the urine sample was sampled by means of a flow reserving valve controlled by the central control unit 15 allowing the accurate sampling of 20 ml of sample. The 20 ml urine sample was placed into the sampling vessel 7, while in the lack of the need of additional sampling the remaining urine was disposed by closing the valve and was discharged from the device through the drain pipe by using known methods available also from the prior art. After the measurement, the device was flushed with a liquid that provides good hygiene, such as clear water or a liquid containing a disinfectant.

Basically, the urine in the plastic sampling vessel 7 is a transparent liquid having various shades of yellow. In order to eliminate the turbidity or incomplete transparency of the sample, it was filtered by a filter placed in the sampling vessel 7 and was allowed to sediment for a few minutes before the analysis. After sampling, the sampling vessel 7 was moved forward on a horizontal conveyor belt in three determined positions:
1. the sampling vessel 7 was placed under the urine dosing tube and 20 ml of clear urine was introduced into the sampling vessel 7.
2. at the second position [measurement point], the dropping of the reagent solution was started from the reagent solution storage unit 3 according to a predetermined protocol corresponding to the selected analysis, as a result of which the change in the turbidity of the urine was monitored by the turbidity meter placed in the turbidity measuring unit 2.
3. at the third, last position, the content of the sampling vessel 7 filled with urine was placed into the disposal container 20.

According to the commands of the central control unit 15, for the detection of anions and/or cations, like iodide ions in this case by selective precipitation reaction, 0,5 mol/l Cu(NO₃)₂ solution was added dropwise by means of a pump 4 from the reagent solution storage unit 3 until otherwise clear urine began to opalesce and a brownish cloudy precipitate was observed and was determined by means of the turbidity meter in the turbidity measuring unit 2. After each drop the urine sample was shaken mechanically and there was a waiting tine of 10 seconds before the next drop was added. Already after the first drop has been added, a colour change occured and the originally yellow urine started to turn green. The color change did not affect the measurement, only the formation of a cloudy, brownish precipitate is measured by the turbidity meter. After the urine began to cloud, but the precipitate had not yet precipitate, one single drop was needed for detecting the precipitate, which indicated the end of the measurement. The iodide ion concentration in the urine is given by the number of the drops after which a brownish cloudy precipitate was observed. The more drops needed, the less iodide ions were secreted by the body. In this case, the reagent solution is added to the urine by an automated pump 4. The central control unit 15 transmitted the measurement results, i.e. the number of droplets used to the unit for collecting and transmitting data 6 through a data transmitting channel. The unit collecting and transmitting data 6 associated the obtained measurement result with the assigned ioFIT identifier at the beginning of the measurement; during data transmission data was also sent to the Rz accounter which charged the same ioFIT Rz account [smart contract]. Upon transmitting the parameter [drop number] presenting the result of the analysis to the IT device with the ioFIT identifier [simultaneously saving the data in the IT device], the account of the same ioFIT identifier was automatically debited with an [Rz] internal accounting unit amount predefined in the smart contract [which was credited to the MID account of the party performing the measurement], and thereby the contract was executed.

The analysis of the precipitate showed that the solution contained CuI as a precipitate. Therefore, the copper nitrate solution was suitable for the selective detection of iodide ions in urine.

The evaluation can be carried out according to Table 1 below.

**Table 1.: Correlation of iodide ion concentration ranges and the drop number of the suitably selected reagent solution.**

| Iodide ion concentration | Iodine deficiency status | Solution amount needed for formation of brownish cloudy precipitate |
|---|---|---|
| 〉 99 microgram/liter | optimal | 1-2 drops |
| 50-99 microgram/liter | light iodine deficiency | 3-5 drops |
| 49,8-20 microgram/liter | medium iodine deficiency | 6-7 drops |
| 〈 20 microgram/liter | severe iodine deficiency | over 8 drops |

### 3. Reference example for detecting iodide ion concentration

During calibration of the drop numbers the concentration of the iodide ions of the same urine sample was determined in mg/L with ICP-MS method, in five parallel measurements, on basis of which the theoretical amount of the copper solution was calculated on basis of the solubility product, thereby determining the necessary concentration. By the copper concentration used, the consumption was proportional to the concentration of the iodide ions. The fact that the precipitate was copper(I) iodide was acknowledged by ICP-MS.

### REFERENCE SIGNS LIST

- 1: sampling unit
- 2: turbidity measuring unit
- 3: reagent solution storage unit
- 4: pump
- 5: human machine interface (HMI)
- 6: unit for collecting and transmitting data
- 7: sampling vessel
- 10: sampling vessel storage unit
- 12: dropping needle
- 13: horizontal sample forwarding unit
- 14: vertical sample forwarding unit
- 15: control unit
- 16: housing
- 20: disposal vessel

## Claims

1. A device for automatic determination of anion and/or cation levels in urine, which comprises:
a) at least one sampling unit(1) and sampling vessels (7), wherein said sampling unit is configured to sample one or more samples of urine into said sampling vessels,
b) at least one reagent solution storage unit (3) comprising a reagent solution, said reagent solution is suitable for detecting anions and/or cations by selective precipitation reaction,
c) at least a pump (4) for delivering a reagent solution corresponding to the precipitation reaction with the specific ion,
d) the sampling unit contains at least one electromechanical unit enabling the horizontal and vertical movements of the sampling vessels;
e) at least one unit for collecting and transmitting data (6),
f) at least one control unit (15),
g) a human machine interface (HMI) and
h) a housing (16), the housing (16) being mounted to a vertical supporting structure, and enclosing said units and being designed for urine collection, wherein the design of the housing is a urinal design or toilet, wherein the device further comprises at least one turbidity measuring unit (2), and a unit fitted with dropping needle (12) wherein the at least one reagent solution storage unit (3) contains reagents selected from the group consisting of:
- sodium hydroxide for detecting magnesium ions,
- sodium hexanitrito cobaltate(III) for detecting potassium ions,
- potassium hexahydroxy antimonate[V] for detecting sodium ions,
- potassium hydrogen phosphate for detecting lithium ions,
- copper nitrate for detecting iodide ions,
- hydrochloric acid medium with thiourea for detecting selenate ions, and
- lithium hydroxide for detecting phosphate ions; and the device control unit (15) is configured to command the pump (4) to add the reagent to the urine in a sampling vessel drop by drop through the dropping needle (12) until the turbidity cause by the precipitation is detected by the turbidity measuring unit (2), wherein the concentration a particular cation or anion is proportional to the number of drops of the respective reagent.

2. The device according to claim 1, wherein the turbidity measuring unit (2) comprises at least one turbidity measuring detector unit with a UV, IR or IR-LED light source.

3. The device according to claim 2 further comprising measuring means for determining the temperature, conductivity and pH of the urine, preferably a ISFET pH sensor or a temperature sensor.

4. The device according to any of claims 1 to 3 wherein the reagent solution storage unit (3) contains the following reagent solutions: sodium hydroxide, sodium hexanitrito cobaltate[III], potassium hexahydroxy antimonate[V], potassium hydrogen phosphate, copper nitrate, thiourea dissolved in hydrochloric acid medium, and lithium hydroxide.

5. The device according to any of claims 1 to 4 wherein one or more urine anions and/or cations or other urine parameters can be measured simultaneously.

6. The device according to any of claims 1 to 5 wherein the turbidity sensor is in connection with the sampling vessel storage unit (10) through the horizontal and vertical sample forwarding unit, which are arranged under common logical control with the sampling vessel storage unit (10), wherein said control includes set of IT elements for computing the measurement result i.e. the number of the droplets of the used reagent solution, and elements transmitting the same to the unit for collecting and transmitting data (6).

7. The device according to any of claims 1 to 6 wherein the reagent solution are introduced into the sampling vessel (7) via a unit fitted with a dropping needle (12).

8. The device according to any of claims 1 to 7 wherein the sampling unit (1) comprises a flow detector and flow meter for separating 1-100 ml of urine, preferably 10-20 ml of urine into a sampling vessel (7).

9. The device according to any of claims 1 to 8 further comprising, within its active range, an adjustable inner parameter storing unit and a built-in radio frequency communication transceiver for the detection of the proximity of IT devices, preferably smart devices having the similarly configured parameters and an identifier.

10. The device according to any of claims 9 further comprising a radio frequency transceiver, an antenna, a mechanical unit for fixing the said device, wherein the active range of the radio frequency transceiver can be variably set to specific values, and the inner parameter storage unit has a fix selection template preset to the to the parameters stored in it, wherein said selection template can be broadened or modified.

11. The device according to any of claims 1 to 10 having a digital accounting system associated with measurements for offsetting each test service in which, when a measurement is made, the transaction value in internal accounting unit is subtracted from the internal accounting units of the smart device having the same identifier.

12. Use of the device according to any of the claims 1 to 11 for automated determination of anion and/or cation levels in urine, comprising the following steps:
a) providing a device according to any of claims 1 to 11,
b) selecting the ion to be measured in the urine through the human-machine interface or the IT device being in connection with the device by the subject needing the analysis,
c) urine sampling in the sampling unit of the device after urination,
d) adding a reagent solution suitable for the detection of the selected ion forwarded by the pump drop by drop to the sample of a predetermined volume taken from the urine to be analyzed, until the turbidity caused by the precipitation is detected,
e) recording the data of drop number of the reagent solution being proportional with the anion and/or cation concentration of the urine, said data being recorded in the unit for collecting and transmitting data, and
f) calculating the concentration data by the device and/or on the smart device of the subject,
wherein the anion and/or cation detected in the urine is selected from Na⁺, K⁺, Mg²⁺, I⁻, Se²⁻, Li⁺, PO₄³⁻ ions, and wherein the precipitation is caused by reagents other than enzymes.

13. The use according to claim 12 wherein the reagent solutions suitable for detecting anions and/or cations through selective precipitation reaction are the following: sodium hydroxide, sodium hexanitrito cobaltate[III], potassium hexahydroxy antimonate[V], potassium hydrogen phosphate, copper nitrate, thiourea dissolved in hydrochloric acidic medium, and lithium hydroxide.

14. The use according to any of the claims 12 or 13, wherein from the analysis of the sequential urine samples the given anion and/or cation levels can be monitored continuously for more days.

15. The use according to any of the claims 12 to 14 for determining the necessity of intake or abandonment of certain anions and/or cations, allowing thereby for the prevention of certain diseases.

## Patentansprüche

1. Vorrichtung zur automatischen Bestimmung von Anionen- und/oder Kationenwerten in Urin, die umfasst:
a) wenigstens eine Probenahmeeinheit (1) und Probenahmebehälter (7), wobei die Probenahmeeinheit dafür ausgelegt ist, eine oder mehrere Urinproben in die Probenahmebehälter zu entnehmen,
b) wenigstens eine Reagenzlösung-Speichereinheit (3), die eine Reagenzlösung umfasst, wobei die Reagenzlösung zum Nachweisen von Anionen und/oder Kationen durch eine selektive Fällungsreaktion geeignet ist,
c) wenigstens eine Pumpe (4) zum Zuführen einer Reagenzlösung entsprechend der Fällungsreaktion mit dem spezifischen Ion,
d) die Probenahmeeinheit enthält wenigstens eine elektromechanische Einheit, die die horizontale und vertikale Bewegung der Probenahmebehälter ermöglicht;
e) wenigstens eine Einheit zum Sammeln und Übertragen von Daten (6),
f) wenigstens eine Steuereinheit (15),
g) eine Mensch-Maschine-Schnittstelle (HMI, Human Machine Interface) und
h) ein Gehäuse (16), wobei das Gehäuse (16) an einer vertikalen Tragstruktur angebracht ist und die Einheiten umschließt und zur Urinsammlung ausgestaltet ist, wobei die Ausgestaltung des Gehäuses die Form eines Urinals oder einer Toilette hat, wobei die Vorrichtung ferner wenigstens eine Trübungsmesseinheit (2) und eine mit einer Tropfennadel (12) versehene Einheit umfasst,
wobei die wenigstens eine Reagenzlösungs-Speichereinheit (3) Reagenzien enthält, die ausgewählt sind aus der Gruppe bestehend aus:
- Natriumhydroxid zum Nachweis von Magnesiumionen,
- Natriumhexanitritokobaltat(III) zum Nachweis von Kaliumionen,
- Kaliumhexahydroxyantimonat[V] zum Nachweis von Natriumionen,
- Kaliumhydrogenphosphat zum Nachweis von Lithiumionen,
- Kupfernitrat zum Nachweis von Iodidionen,
- Salzsäuremedium mit Thioharnstoff zum Nachweis von Selenationen und
- Lithiumhydroxid zum Nachweis von Phosphationen;
und die Vorrichtung wobei die Steuereinheit (15) dafür ausgelegt ist, die Pumpe (4) so zu steuern, dass sie das Reagenz tropfenweise durch die Tropfennadel (12) in den Urinprobenahmebehälter pumpt, bis die durch die Fällung verursachte Trübung von der Trübungsmesseinheit (2) erfasst wird, wobei die Konzentration eines bestimmten Kations oder Anions proportional zur Anzahl der Tropfen des jeweiligen Reagenzes ist.

2. Vorrichtung gemäß Anspruch 1, wobei die Trübungsmesseinheit (2) wenigstens eine Trübungsmessdetektoreinheit mit einer UV-, IR- oder IR-LED-Lichtquelle umfasst.

3. Vorrichtung gemäß Anspruch 2, ferner Messmittel zum Bestimmen der Temperatur, der Leitfähigkeit und des pH-Wertes des Urins umfassend, vorzugsweise einen ISFET-pH-Sensor oder einen Temperatursensor.

4. Vorrichtung gemäß einem der Ansprüche 1 bis 3, wobei die Reagenzlösungs-Speichereinheit (3) die folgenden Reagenzlösungen enthält: Natriumhydroxid, Natriumhexanitritokobaltat[III], Kaliumhexahydroxyantimonat[V], Kaliumhydrogenphosphat, Kupfernitrat, in Salzsäuremedium gelösten Thioharnstoff und Lithiumhydroxid.

5. Vorrichtung gemäß einem der Ansprüche 1 bis 4, wobei ein oder mehrere Anionen und/oder Kationen des Urins oder andere Parameter des Urins gleichzeitig gemessen werden können.

6. Vorrichtung gemäß einem der Ansprüche 1 bis 5, wobei der Trübungssensor mit der Probenahmebehälter-Speichereinheit (10) über die horizontale und die vertikale Probenweiterleitungseinheit, die unter gemeinsamer logischer Steuerung mit der Probenahmebehälter-Speichereinheit (10) angeordnet sind, verbunden ist, wobei die Steuerung eine Reihe von IT-Elementen zum Berechnen des Messergebnisses, d. h. der Anzahl der Tropfen der verwendeten Reagenzlösung, und Elemente, die diese an die Einheit zum Sammeln und Übertragen von Daten (6) übertragen, umfasst.

7. Vorrichtung gemäß einem der Ansprüche 1 bis 6, wobei die Reagenzlösung über eine mit einer Tropfennadel (12) versehene Einheit in den Probenahmebehälter (7) zugeführt wird.

8. Vorrichtung gemäß einem der Ansprüche 1 bis 7, wobei die Probenahmeeinheit (1) einen Durchflussdetektor und einen Durchflussmesser umfasst, zum Abscheiden von 1-100 ml Urin, vorzugsweise 10-20 ml Urin, in einen Probenahmebehälter (7).

9. Vorrichtung gemäß einem der Ansprüche 1 bis 8, ferner umfassend, innerhalb ihres Wirkbereichs, eine einstellbare innere Parameterspeichereinheit und einen eingebauten Sendeempfänger für Hochfrequenz-Kommunikation zum Erkennen der Nähe von IT-Vorrichtungen, vorzugsweise intelligenten Vorrichtungen mit den ähnlich konfigurierten Parametern und einer Kennung.

10. Vorrichtung gemäß einem der Ansprüche 9, ferner einen Hochfrequenz-Sendeempfänger, eine Antenne, eine mechanische Einheit zur Befestigung der Vorrichtung umfassend, wobei die Reichweite des Hochfrequenz-Sendeempfängers variabel auf spezifische Werte einstellbar ist und die innere Parameterspeichereinheit eine auf die darin gespeicherten Parameter voreingestellte Auswahlschablone aufweist, wobei die Auswahlschablone erweiterbar oder veränderbar ist.

11. Vorrichtung gemäß einem der Ansprüche 1 bis 10 mit einem digitalen Abrechnungssystem, das mit Messungen zum Verrechnen jedes Testdienstes verbunden ist, wobei bei einer Messung der Transaktionswert in der internen Abrechnungseinheit von den internen Abrechnungseinheiten der intelligenten Vorrichtung mit derselben Kennung abgezogen wird.

12. Verwendung der Vorrichtung gemäß einem der Ansprüche 1 bis 11 zur automatisierten Bestimmung von Anionen- und/oder Kationenwerten in Urin, die folgenden Schritte umfassend:
a) Bereitstellen einer Vorrichtung gemäß einem der Ansprüche 1 bis 11,
b) Auswählen des im Urin zu messenden Ions über die Mensch-Maschine-Schnittstelle oder die IT-Vorrichtung, die mit der Vorrichtung verbunden ist, durch die Person, die die Analyse benötigt,
c) Urinprobenentnahme in der Probenahmeeinheit der Vorrichtung nach dem Urinieren,
d) Hinzufügen einer für den Nachweis des ausgewählten Ions geeigneten Reagenzlösung, die von der Pumpe tropfenweise in die Probe eines vorbestimmten Volumens, die aus dem zu analysierenden Urin entnommen wurde, weitergeleitet wird, bis die durch die Fällung verursachte Trübung nachgewiesen wird,
e) Aufzeichnen der Daten zur Anzahl der Tropfen der Reagenzlösung, die proportional zu der Konzentration von Anionen und/oder Kationen im Urin ist, wobei die Daten in der Einheit zum Sammeln und Übertragen von Daten aufgezeichnet werden, und
f) Berechnen der Konzentrationsdaten durch die Vorrichtung und/oder auf der intelligenten Vorrichtung der Person,
wobei das im Urin nachgewiesene Anion und/oder Kation aus Na⁺-, K⁺-, Mg²⁺-, I⁻-, Se²⁻-, Li⁺-, PO₄³⁻-Ionen ausgewählt ist und wobei die Fällung durch andere Reagenzien als Enzyme verursacht wird.

13. Verwendung gemäß Anspruch 12, wobei die Reagenzlösungen, die zum Nachweisen von Anionen und/oder Kationen durch selektive Fällungsreaktion geeignet sind, die folgenden sind: Natriumhydroxid, Natriumhexanitritokobaltat[III], Kaliumhexahydroxyantimonat[V], Kaliumhydrogenphosphat, Kupfernitrat, in Salzsäuremedium gelöster Thioharnstoff, und Lithiumhydroxid.

14. Verwendung gemäß einem der Ansprüche 12 oder 13, wobei durch die Analyse der aufeinanderfolgenden Urinproben die gegebenen Anionen- und/oder Kationenwerte kontinuierlich über mehrere Tage überwacht werden können.

15. Verwendung gemäß einem der Ansprüche 12 bis 14 zum Bestimmen der Notwendigkeit der Einnahme oder des Verzichts auf bestimmte Anionen und/oder Kationen, wodurch die Prävention bestimmter Krankheiten ermöglicht wird.

## Revendications

1. Dispositif pour la détermination automatique de niveaux d'anions et/ou de cations dans l'urine, comprenant:
a) au moins une unité d'échantillonnage (1) et des récipients d'échantillonnage (7), ladite unité d'échantillonnage étant configurée pour prélever un ou plusieurs échantillons d'urine dans lesdits récipients d'échantillonnage,
b) au moins une unité de stockage de solution de réactif (3) comprenant une solution de réactif, ladite solution de réactif convenant à la détection d'anions et/ou de cations par réaction de précipitation sélective,
c) au moins une pompe (4) pour la distribution d'une solution de réactif correspondant à la réaction de précipitation avec l'ion spécifique,
d) l'unité d'échantillonnage contenant au moins une unité électromécanique permettant les mouvements horizontaux et verticaux des récipients d'échantillonnage;
e) au moins une unité de collecte et de transmission de données (6),
f) au moins une unité de commande (15),
g) une interface homme-machine (HMI) et
h) un boîtier (16), le boîtier (16) étant monté sur une structure de support verticale, entourant lesdites unités et conçu pour la collecte d'urine, le boîtier ayant une conception de type urinoir ou toilette, le dispositif comprenant en outre au moins une unité de mesure de turbidité (2) et une unité munie d'une aiguille de gouttage (12),
l'au moins une unité de stockage de solution de réactif (3) contenant des réactifs sélectionnés dans le groupe constitué de :
- l'hydroxyde de sodium pour la détection des ions magnésium,
- l'hexanitritocobaltate (III) de sodium pour la détection des ions potassium,
- l'antimonate[V] hexahydroxyde de potassium pour la détection des ions sodium,
- le phosphate monohydrogéné de potassium pour la détection des ions lithium,
- le nitrate de cuivre pour la détection des ions iodure,
- un milieu d'acide chlorhydrique avec de la thiourée pour la détection des ions sélénate, et
- l'hydroxyde de lithium pour la détection des ions phosphate ;
et le dispositif l'unité de commande (15) étant conçue pour commander la pompe (4) afin d'ajouter le réactif à l'urine dans un récipient d'échantillonnage goutte à goutte par l'intermédiaire de l'aiguille de gouttage (12) jusqu'à ce que la turbidité causée par la précipitation soit détectée par l'unité de mesure de turbidité (2), la concentration d'un cation ou d'un anion particulier étant proportionnelle au nombre de gouttes du réactif correspondant.

2. Dispositif selon la revendication 1, l'unité de mesure de turbidité (2) comprenant au moins une unité de détection de mesure de turbidité dotée d'une source de lumière UV, IR ou IR-LED.

3. Dispositif selon la revendication 2 comprenant en outre des moyens de mesure destinés à déterminer la température, la conductivité et le pH de l'urine, de préférence un capteur de pH ISFET ou un capteur de température.

4. Dispositif selon l'une quelconque des revendications 1 à 3, l'unité de stockage de solution de réactif (3) contenant les solutions de réactif suivantes: hydroxyde de sodium, hexanitritocobaltate (III) de sodium, antimonate[V] hexahydroxyde de potassium, phosphate monohydrogéné de potassium, nitrate de cuivre, thiourée dissoute dans un milieu d'acide chlorhydrique et hydroxyde de lithium.

5. Dispositif selon l'une quelconque des revendications 1 à 4, un ou plusieurs anions et/ou cations urinaires ou d'autres paramètres urinaires pouvant être mesurés simultanément.

6. Dispositif selon l'une quelconque des revendications 1 à 5, le capteur de turbidité étant en connexion avec l'unité de stockage de récipients d'échantillonnage (10) au moyen de l'unité de transfert d'échantillons horizontale et verticale, celles-ci étant placées sous un contrôle logique commun avec l'unité de stockage de récipients d'échantillonnage (10), ledit contrôle comprenant un ensemble d'éléments informatiques pour calculer le résultat de mesure, c'est-à-dire le nombre de gouttes de la solution de réactif utilisée, et des éléments transmettant celui-ci à l'unité de collecte et de transmission de données (6).

7. Dispositif selon l'une quelconque des revendications 1 à 6, la solution de réactif étant introduite dans le récipient d'échantillonnage (7) par l'intermédiaire d'une unité équipée d'une aiguille de gouttage (12).

8. Dispositif selon l'une quelconque des revendications 1 à 7, l'unité d'échantillonnage (1) comprenant un détecteur de débit et un débitmètre pour séparer 1 à 100 ml d'urine, de préférence 10 à 20 ml d'urine, dans un récipient d'échantillonnage (7).

9. Dispositif selon l'une quelconque des revendications 1 à 8 comprenant en outre, dans sa plage active, une unité interne de stockage de paramètres réglables et un émetteur-récepteur de communication à radiofréquence intégré pour la détection de la proximité de dispositifs informatiques, de préférence des dispositifs intelligents ayant les mêmes paramètres configurés et un identifiant.

10. Dispositif selon la revendication 9 comprenant en outre un émetteur-récepteur radiofréquence, une antenne, une unité mécanique pour la fixation dudit dispositif, la plage active de l'émetteur-récepteur radiofréquence pouvant être réglée de manière variable à des valeurs spécifiques, et l'unité interne de stockage de paramètres disposant d'un gabarit de sélection fixe préréglé sur les paramètres qui y sont stockés, ledit gabarit de sélection pouvant être élargi ou modifié.

11. Dispositif selon l'une quelconque des revendications 1 à 10 doté d'un système de comptabilité numérique associé aux mesures pour l'imputation de chaque prestation de test, dans lequel, lorsqu'une mesure est effectuée, la valeur de transaction en unité de comptabilité interne est soustraite des unités de comptabilité internes du dispositif intelligent ayant le même identifiant.

12. Utilisation du dispositif selon l'une quelconque des revendications 1 à 11 pour la détermination automatisée de niveaux d'anions et/ou de cations dans l'urine, comprenant les étapes suivantes:
a) la fourniture d'un dispositif selon l'une quelconque des revendications 1 à 11,
b) la sélection de l'ion devant être mesuré dans l'urine par l'intermédiaire de l'interface homme-machine ou du dispositif informatique connecté audit dispositif par le sujet nécessitant l'analyse,
c) le prélèvement d'urine dans l'unité d'échantillonnage du dispositif après la miction,
d) l'ajout d'une solution de réactif appropriée pour la détection de l'ion sélectionné, acheminée par la pompe goutte à goutte au prélèvement d'un volume prédéterminé prélevé dans l'urine à analyser, jusqu'à ce que la turbidité provoquée par la précipitation soit détectée,
e) l'enregistrement des données du nombre de gouttes de la solution de réactif étant proportionnel à la concentration en anions et/ou cations de l'urine, lesdites données étant enregistrées dans l'unité de collecte et de transmission de données, et
f) le calcul des données de concentration par le dispositif et/ou sur le dispositif intelligent du sujet, les anions et/ou cations détectés dans l'urine étant sélectionnés parmi les ions Na⁺, K⁺, Mg²⁺, I⁻, Se²⁻, Li⁺, PO₄³⁻, et la précipitation étant provoquée par des réactifs autres que des enzymes.

13. Utilisation selon la revendication 12, les solutions de réactif adaptées à la détection d'anions et/ou de cations par réaction de précipitation sélective étant les suivantes : hydroxyde de sodium, hexanitritocobaltate (III) de sodium, antimonate[V] hexahydroxyde de potassium, phosphate monohydrogéné de potassium, nitrate de cuivre, thiourée dissoute dans un milieu d'acide chlorhydrique et hydroxyde de lithium.

14. Utilisation selon l'une quelconque des revendications 12 ou 13, l'analyse des échantillons d'urine séquentiels permettant de surveiller en continu les niveaux d'anions et/ou de cations concernés pendant plusieurs jours.

15. Utilisation selon l'une quelconque des revendications 12 à 14 pour déterminer la nécessité de l'apport ou de l'abandon de certains anions et/ou cations, permettant ainsi la prévention de certaines maladies.
